# EUROPÄISCHE PATENTANMELDUNG

(11) **EP 0 943 917 A2**
(43) Veröffentlichungstag der Anmeldung: **22.09.1999**
(21) Anmeldenummer: 99104043.7
(22) Anmeldetag: 16.03.1999
(51) Int. Cl.: G01N 29/02

(54) **Modular aufgebautes Gassensorgerät mit einer Vielzahl polymerbeschichteter Oberflächenwellen(SAW)-Resonatoren**

(30) Priorität: 17.03.1998 DE 29804805 U
(71) Anmelder: Bürkert Werke GmbH & Co., D-74653 Ingelfingen (DE)
(72) Erfinder: Töpfer, Heinz, Prof. Dr., 01277 Dresden (DE)
(74) Vertreter: Degwert, Hartmut, Dipl.-Phys.

(57) **Zusammenfassung**

Ein Gassensorgerät enthält einen Sensorkopf (10), der mit gassensitiven Polymeren beschichtete Oberflächenwellen-Resonatoren aus piezoelektrischem Keramik- bzw. Kristallmaterial aufweist, mit einer elektronischen Steuer- und Auswerteeinheit und mit dem Sensorkopf (10) zugeordneten Fluidikeinheiten für Beprobung und Spülung. Das Gerät hat einen modularen Aufbau, worin die folgenden Komponenten austauschbar miteinander kombinierbar sind:
- der Sensorkopf (10),
- eine Auswerte- und Anzeigeeinheit (12),
- die Steuereinheit (20) für die Steuerung der Meßabläufe,
- eine interne Fluidikeinheit (14),
- eine externe Fluidikeinheit (16) und
- eine Steuereinheit (18) für wenigstens einer der beiden Fluidikeinheiten.

## Beschreibung

Die Erfindung betrifft ein Gassensorgerät mit einem Sensorkopf, der mit gassensitiven Polymeren beschichtete Oberflächenwellen-Resonatoren aus piezoelektrischem Keramik- oder Kristallmaterial aufweist, mit einer elektronischen Steuer- und Auswerteeinheit und mit dem Sensorkopf zugeordneten Fluidikeinheiten für Beprobung und Spülung.

Bei einem solchen Gassensor wird in aufeinanderfolgenden Zyklen abwechselnd z.B. Luft bzw. Meßgas aus einer überwachten Umgebung und ein neutrales Spülgas durch Fluidikeinheiten dem Sensorkopf zugeführt. An den gassensitiven Polymeren, mit denen die Oberflächenwellen-Resonatoren beschichtet sind, findet eine Anlagerung (Sorption) von Gasen statt, die zu einer meßbaren Veränderung der Resonanzfrequenz der erzeugten Oberflächenwellen führt. Durch Mischung mit dem Signal eines Referenzoszillators entsteht eine Differenzfrequenz, die gemessen und ausgewertet werden kann. Die Differenzfrequenz ist für jede Kombination von Polymerschicht und Meßgas charakteristisch. In dem Sensorkopf sind mehrere Oberflächenwellen-Resonatoren mit verschiedenen Beschichtungen zusammengefaßt. Die Signale der verschiedenen Oberflächenwellen-Resonatoren werden kombiniert und zu einem Signalmuster verarbeitet. Die resultierenden charakteristischen Signalmuster sind durch die Art des gemessenen Gases und durch die Zusammenstellung der verwendeten Polymerschichten bestimmt. Die Auswertung der Signalmuster kann mit neuronalen Netzen erfolgen.

Üblicherweise wird ein solches Gassensorgerät komplett in einem Gehäuse aufgebaut, das sämtliche Bestandteile einschließlich der Fluidikeinheiten aufnimmt. Das zu überwachende Gas wird über eine Schlauchleitung an das Gerät herangeführt. Je nach Einsatzzweck und
-ort werden unterschiedliche Anforderungen an das Gerät gestellt. Während beispielsweise in einem Labor eine hohe Genauigkeit gefordert wird, die eine besondere Thermostatisierung des Sensorkopfes notwendig macht, ist beim Einsatz vor Ort oder in einer Warte eine autonome Energieversorgung erwünscht oder notwendig. Für den Einsatz in explosionsgefährdeten Umgebungen sind besondere Sicherheitsmaßnahmen erforderlich, die zusätzlichen Aufwand erfordern. Eine Ausführung des Gassensorgeräts, die allen Anforderungen genügt, wäre zu aufwendig. Es werden daher verschiedene Geräteausführungen für unterschiedliche Einsatzorte und -zwecke produziert.

Durch die Erfindung wird der Aufwand für eine anwendungsoptimierte Ausführung des Gassensorgeräts erheblich vermindert. Gemäß der Erfindung ist das Gassensorgerät durch einen modularen Aufbau gekennzeichnet, worin die folgenden Komponenten austauschbar miteinander kombinierbar sind:
- der Sensorkopf,
- eine Auswerte- und Anzeigeeinheit,
- eine Steuereinheit für die Steuerung der Meßabläufe,
- eine interne Fluidikeinheit,
- eine externe Fluidikeinheit und
- eine Steuereinheit für wenigstens eine der beiden Fluidikeinheiten.

Durch die modulare Ausführung des erfindungsgemäßen Gassensorgeräts können seine Komponenten einzeln für die gewünschten Einsatzbedingungen optimiert werden. Vorzugsweise werden standardisierte Schnittstellen zwischen den Komponenten des Geräts verwendet, so daß der Austausch erleichtert wird. Ein bestehendes Gerät kann durch Auswechseln einer Komponente leicht auf- oder abgerüstet werden. Wartung und Instandsetzung werden durch den modularen Aufbau ebenfalls erleichtert.

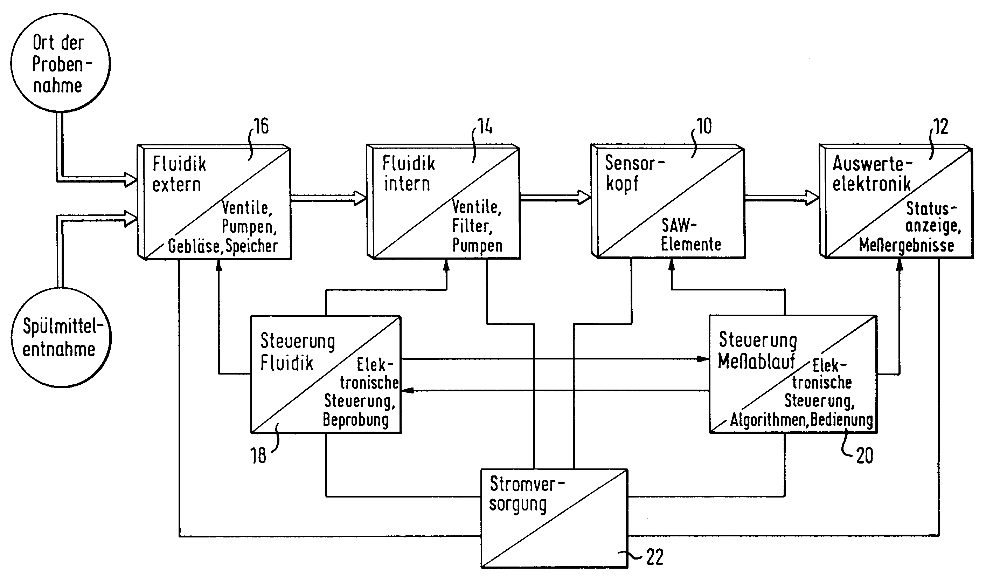

Weitere Merkmale und Vorteile der Erfindung ergeben sich aus der folgenden Beschreibung einer Ausführungsform des Gassensorgeräts unter Bezugnahme auf die Zeichnung. Die einzige Figur der Zeichnung zeigt das Gassensorgerät in einem Blockschaltbild.

Das erfindungsgemäße Gassensorgerät wird modular aus den in der Zeichnung dargestellten Komponenten aufgebaut. Ein Sensorkopf 10 enthält beispielsweise acht unterschiedlich beschichtete Oberflächenwellen-Resonatoren mit zugehöriger HF-Elektronik einschließlich Thermostatisierung. Das von dem Sensorkopf 10 erzeugte Signalmuster gelangt zu einer Auswerte- und Anzeigeeinheit 12. Diese enthält einen Mikrocomputer für die Datenerfassung und -auswertung. Eine interne Fluidikeinheit 14 ist mit dem Sensorkopf 10 durch eine lösbare Verbindung oder dergleichen gekoppelt. Sie beinhaltet Ventile, Filter, Pumpen und dergleichen. Eine externe Fluidikeinheit 16 ist über die interne Fluidikeinheit 14 mit dem Sensorkopf 10 gekoppelt. Auch diese Fluidikeinheit enthält Ventile, Pumpen, Gebläse oder dergleichen, je nach Verwendungszweck des Gassensorgeräts. Über eine Schlauchleitung oder dergleichen ist die externe Fluidikeinheit 16 mit dem Ort der Probenahme sowie mit einer Spülgasentnahme gekoppelt. Die Funktionen der Fluidikeinheiten 14, 16 werden durch eine Steuereinheit 18 gesteuert. Durch diese Steuereinheit 18 wird der Gasaustausch am Sensorkopf 10 zwischen Meßgas in der Meßphase und Spülgas in der Spülphase gesteuert und überwacht. Eine weitere Steuereinheit 20 ist für die Steuerung des Meßablaufs vorgesehen und mit dem Sensorkopf 10 sowie mit der Auswerteelektronik 12 gekoppelt. Die Steuereinheiten 18 und 20 sind auch miteinander gekoppelt. Alle Komponenten des Gassensorgeräts werden von einem gemeinsamen Stromversorgungsblock 22 gespeist.

Alle beschriebenen Komponenten verfügen über definierte Schnittstellen und sind daher leicht austauschbar. Jede einzelne Komponente kann für den gewünschten Einsatzzweck konfiguriert und optimiert sein.

## Patentansprüche

1. Gassensorgerät mit einem Sensorkopf (10), der mit gassensitiven Polymeren beschichtete Oberflächenwellen-Resonatoren aus piezoelektrischem Keramik- bzw. Kristallmaterial aufweist, mit einer elektronischen Steuer- und Auswerteeinheit und mit dem Sensorkopf (10) zugeordneten Fluidikeinheiten für Beprobung und Spülung, gekennzeichnet durch einen modularen Aufbau, worin die folgenden Komponenten austauschbar miteinander kombinierbar sind:
- der Sensorkopf (10),
- eine Auswerte- und Anzeigeeinheit (12),
- eine Steuereinheit (20) für die Steuerung der Meßabläufe,
- eine interne Fluidikeinheit (14),
- eine externe Fluidikeinheit (16) und
- eine Steuereinheit (18) für wenigstens einer der beiden Fluidikeinheiten.

2. Gassensorgerät nach Anspruch 1, dadurch gekennzeichnet, daß die Komponenten definierte Schnittstellen aufweisen.

3. Gassensorgerät nach Anspruch 1 oder 2, gekennzeichnet durch modular ausgeführte Energieversorgungs-Komponenten.

4. Gassensorgerät nach einem der vorstehenden Ansprüche, gekennzeichnet durch Kühlungs- und/oder den Meßbedingungen angepaßte Thermostatisierungs-Komponenten.
